# EUROPEAN PATENT SPECIFICATION

(11) **EP 3 863 709 B1**
(45) Date of publication and mention of the grant of the patent: **07.06.2023**
(21) Application number: 19798720.9
(22) Date of filing: 08.10.2019
(51) Int. Cl.: A61N 1/04, A61N 1/36

(54) **ELECTRO-STIMULATION APPARATUS**
ELEKTROSTIMULATIONSGERÄT
APPAREIL D'ÉLECTROSTIMULATION

(30) Priority: 08.10.2018 IT 201800009240
(43) Date of publication of application: 18.08.2021
(73) Proprietor: Wecare Thecnologies S.r.l., 20146 Milan (IT)
(72) Inventor: GENITONI, Valerio, 20146 Milan (IT)
(74) Representative: Di Bernardo, Antonio
(86) International application number: PCT/IB2019/058560
(87) International publication number: WO 2020/075070

(56) References cited:
- US-A1- 2014 031 895
- US-A1- 2014 296 935

## Description

### TECHNICAL FIELD

The present invention relates to an apparatus for electrostimulation. The apparatus finds a preferred application in the treatment of pain syndromes or for implementing an acupuncture therapy electrically.

### BACKGROUND

Electrostimulation apparatuses are used for both acupuncture and transcutaneous electrical nerve stimulation (TENS).

In both cases, electrical signals are applied locally to the patient's body to stimulate particular points in the nervous system.

Traditional acupuncture envisages inserting needles into particular points on the body, called acupuncture points, to stimulate certain nerve lines, called meridians. Given the widespread use of acupuncture, electrical apparatuses have been proposed over time that can stimulate acupuncture points electrically. Examples of electrical apparatuses for acupuncture are described in American patents US 3,900,020, US 7,076,293 and US 6,301,500.

In TENS, electrical signals of different intensity are applied to alleviate pain syndromes. The different intensity of the electrical signals strongly depends on the school of thought followed by the attending physician. A first school of thought, adhering to the Gate Control theory of Melzak & Wall, envisages the use of current intensity in the order of a few MilliAmpere to obtain the blockage of painful information by interfering on the mechanisms of transmission of the neurological pain information. A different school of thought, instead, envisages the use of electrical currents with intensity in the order of the MicroAmpere.

As an example, US 2014/0308195 describes a TENS pain treatment apparatus configured to be worn by a user. Such an apparatus includes a first electrode and a second electrode for providing TENS treatment, and a muscle contraction sensor subsystem configured to detect a muscle contraction profile induced by the electrode array on the patient. Further, a control module is configured to receive an input provided by the muscle contraction sensor subsystem and to modulate a TENS treatment parameter based on that input until a threshold is met.

Furthermore, US 2014/296935 describes an apparatus for transcutaneous electrical nerve stimulation comprising: stimulation means carried by a housing to electrically stimulate at least one nerve through a pair of electrodes; monitoring means to monitor user gestures, electrode-skin contact integrity and a transient movement. Further, the apparatus includes analysis means for analysing the output signal provided by the monitoring means to determine a user gesture, electrode-skin contact integrity and a transient motion, and control means for controlling the output signal of the stimulation means in response to the determined user gesture, electrode-skin contact integrity and a transient motion.

Whether it is electrostimulation apparatuses for acupuncture or TENS, the efficacy of the treatment depends on many factors, including the patient. The human body of different patients, in fact, can present different electrical impedance and, therefore, respond differently to the treatments of electrical stimulation.

For this reason, some electrostimulation apparatuses, such as the one described in US Patent 3,900,020, allow generating electrical signals of different waveforms at the electrodes. The attending physician can thus select the waveform and the frequency s/he deems most appropriate for the patient.

The efficacy of the treatment, however, depends on multiple factors: the experience of the attending physician, the response of the patient, the availability at the level of electrostimulation apparatus of an electrical signal with waveform and intensity ideal for the single patient.

Therefore, the need is felt for electrostimulation apparatuses that are effective on a large sample of patients and easy to use.

### OBJECTS AND SUMMARY OF THE INVENTION

An object of the present invention is to overcome the disadvantages of the prior art.

In particular an object of the present invention is to provide an electrostimulation apparatus capable of applying to the stimulation electrodes a specific current for the treated patient.

An object of the present invention is also to provide an electrostimulation apparatus that is easy to use and effective on a large number of patients.

A further object of the present invention is to provide an electrostimulation method suitable for being implemented through this apparatus.

These and other objects of the present invention are achieved by a system and a method incorporating the features of the accompanying claims, which form an integral part of the present description.

According to a first aspect, the invention is directed to an apparatus for electrostimulation of biological tissues in accordance with appended claim 1.

Thanks to this solution it is possible to modify a chemical-physical condition of the biological tissue to which the electrode is applied. In particular, the identification of a return signal with expected parameters allows optimizing the electrical signal transmitted to the biological tissue by dynamically adapting it to the actual conditions of the biological tissue without requiring invasive instruments and/or dedicated measurement apparatuses. This allows obtaining a particularly effective electrostimulation of biological tissues, in particular, capable of adapting to the peculiarities of a treated subject.

In one embodiment, the control unit is configured for generating the parameters of the electrical signal on the basis of additional parameters comprising at least one among an ambient temperature, an atmospheric pressure, and a time of the day.

In this way it is possible to adapt the control signal so as to compensate effects due to the influence of the environmental conditions on the biological tissue.

In one embodiment, the control unit is configured for generating the parameters of a second electrical signal suitable for bringing a portion of biological tissue onto which an electrode is applied to a predetermined electrical potential. Preferably, the electrical potential is comprised between -30 mV and 0 mV, in the case of antalgic treatments, for example an electrostimulation with micro-currents, while in the case of transdermal electroacupuncture the value of the electrical potential is defined case by case

Thanks to this solution it is possible to easily modify an electrical potential of the portion of biological tissue to which the electrode is applied. Advantageously, the two electrical signals provide a combined treatment which allows implementing particularly effective antalgic and transdermal electroacupuncture treatments.

In one embodiment, the control unit is configured for generating the parameters of a plurality of electrical signals, each of which is transmitted sequentially by a respective electrode, and in which the control unit is configured for generating the parameters of an electrical signal when the parameters of the return electrical signal associated with a previous electrical signal differ from the corresponding parameter of an expected return signal within the threshold value.

This ensures that the signal transmitted to each electrode is optimized before moving on to optimizing the signal on a next electrode. This is particularly effective in the treatment of algic aspects that propagate from a point of origin in the biological tissue.

In one embodiment, the apparatus comprises a user interface suitable for receiving instructions and providing information to an operator of the apparatus. Moreover, the control unit is configured for measuring an electrical potential via the electrode and displaying information based on the electrical potential by means of the electrode through the user interface.

In this way, it is possible to acquire and provide useful information without the need for dedicated analysis and measurement facilities.

In particular, an embodiment envisages that the control unit transmits the electrical potential measurements made by means of at least one electrode to an external unit, such as for example a server. The server processes the measurements and transfers the above information to the control unit. Preferably, such information comprising an indication of one or more electrodes to which the electrical signal is to be applied, identified based on performed electrical potential measurements.

Thanks to this solution it is possible to determine information on the state of the biological tissue analysed by keeping the calculation power of the electronic unit and therefore the cost and consumption thereof limited. Moreover, it is possible to obtain information on the application points of the electrodes that allows obtaining a transdermal electro-acupuncture treatment optimized for the treated subject.

In one embodiment, the control unit is configured for measuring the electrical potential between the electrode and the reference electrode. In this way it is possible to acquire the potential measurements in a differential manner, reducing the complexity of the circuit and eliminating disturbances deriving from a distinct reference terminal from the biological tissue.

In one embodiment, the electrodes comprise probe terminals suitable for contacting the biological tissue. In particular, the probe terminals have a contact surface of extension comprised between 0.5 mm² and 2 mm², when the apparatus is used for transdermal electroacupuncture, or between 1 cm² and 5 cm², when the apparatus is used for antalgic treatments.

In this way it is possible to transmit the electrical signal to a portion of biological tissue suitable for the specific treatment to be implemented. Advantageously, the control unit is configured for adjusting the parameters of the electrical signal on the basis of the dimensions of the probe terminals used.

A different aspect not covered by the claimed invention presents a method for the electrostimulation of biological tissues. This method envisages arranging an apparatus for electrostimulation comprising a signal generator adapted to generate an electrical signal, an electrode electrically connected to the signal generator to receive the electrical signal generated by the signal generator, and a control unit operatively connected to the signal generator to adjust parameters of the generated electrical signal and to read the parameters of an electrical signal returning from the electrode, determining the parameters of an electrical signal, reading the electrical signal returning from the electrode, comparing at least one parameter of the return electrical signal with a corresponding parameter of an expected return signal, and if one or more of the parameters of the return electrical signal differ from the corresponding parameters of the expected return signal beyond a threshold value, then the control unit is configured for modifying the parameters of the electrical signal and repeating the previous steps.

This allows obtaining a particularly effective electrostimulation of biological tissues, in particular, capable of adapting to the peculiarities of a treated subject.

In one embodiment not covered by the claimed invention, the method envisages applying a conductive gel on a probe terminal of the electrode. In particular, the conductive gel comprising an active ingredient suitable for treating an inflammatory or painful condition of a biological tissue.

Thanks to this solution, it is possible to integrate an electrostimulation treatment with the active ingredients contained in the conductive gel. In particular, the electrical signals provided to the biological tissue have the effect of increasing the absorption of these active ingredients contained in the conductive gel in addition to the normal effect on the biological tissue. This synergy between the conductive gel and the electrostimulation allows increasing significantly the efficacy of the treatments implemented, in particular, in the case of antalgic treatments.

Further features and advantages of the present invention will be more apparent from the description of the accompanying drawings.

### BRIEF DESCRIPTION OF THE DRAWINGS

The invention will be described below with reference to some examples, provided for explanatory and non-limiting purposes, and illustrated in the accompanying drawings. These drawings illustrate different aspects and embodiments of the present invention and, where appropriate, reference numerals illustrating similar structures, components, materials and/or elements in different figures are indicated by similar reference numbers.
Figure 1 is a schematic representation of an apparatus for the electrostimulation of biological tissues;
Figure 2 is a functional block diagram of the apparatus of Figure 1 in an operative arrangement;

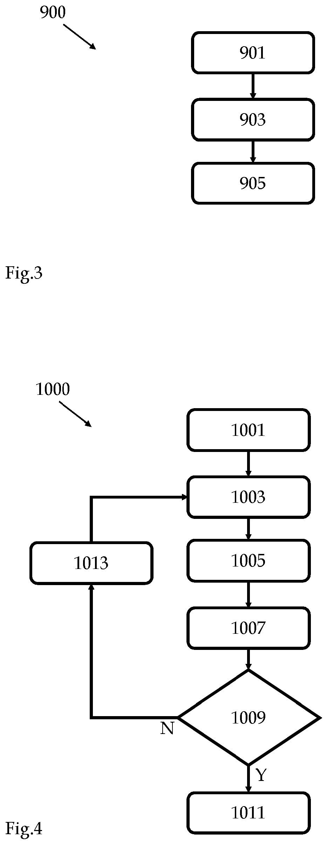

Figures 3 and 4 are flow diagrams relating to procedures for generating and transmitting electrical signals implemented by the apparatus of Figure 1;
Figure 5 shows the trend of electrical signals in an electrode of the apparatus of Figure 1;

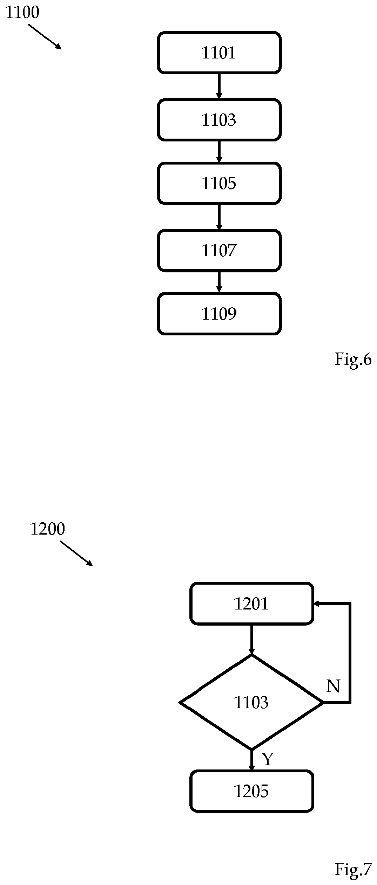

Figure 6 is a flow diagram relating to a measurement procedure implemented by the apparatus of Figure 1;
Figure 7 is a flow diagram relating to a micro-current electrostimulation procedure implemented by the apparatus of Figure 1, and

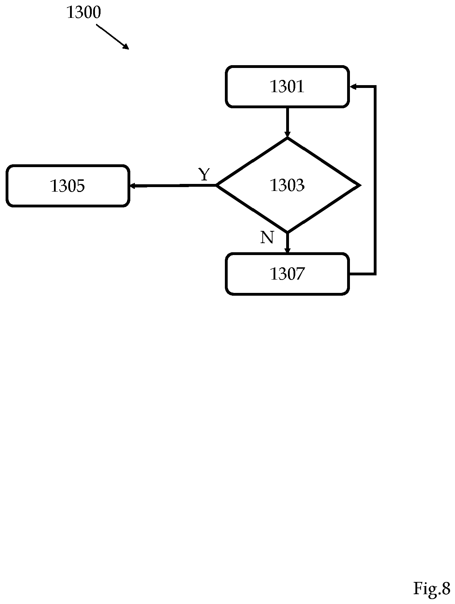

Figure 8 is a flow diagram relating to a transdermal electroacupuncture procedure implemented by the apparatus of Figure 1.

### DETAILED DESCRIPTION OF THE INVENTION

While the invention is susceptible to various modifications and alternative constructions, certain preferred embodiments are shown in the drawings and are described hereinbelow in detail. It is in any case to be noted that there is no intention to limit the invention to the specific embodiment illustrated, rather on the contrary, the invention intends covering all the modifications, alternative and equivalent constructions that fall within the scope of the invention as defined in the claims.

The use of "for example", "etc.", "or" indicates non-exclusive alternatives without limitation, unless otherwise indicated. The use of "includes" means "includes, but not limited to", unless otherwise indicated.

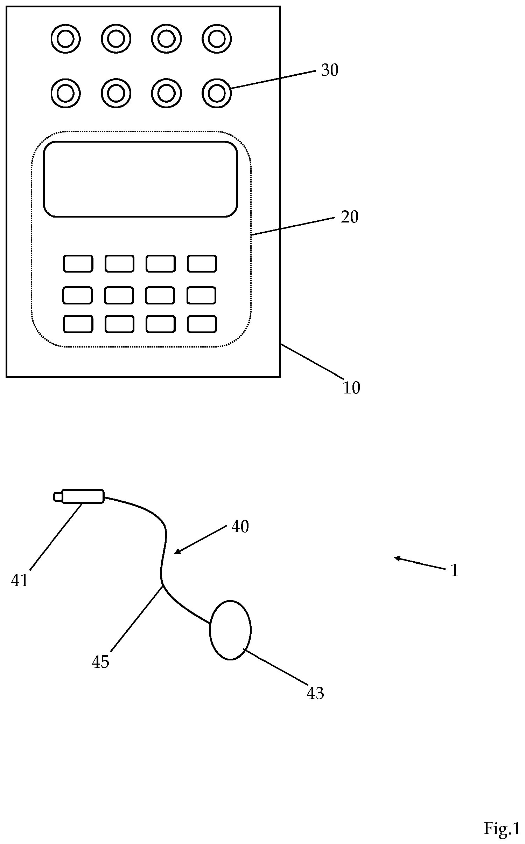

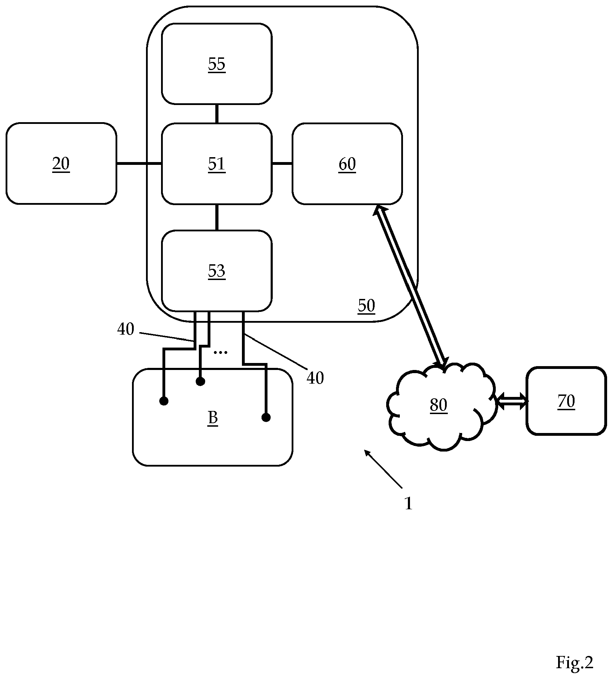

With reference Figures 1 and 2 an apparatus 1 for the electrostimulation of biological tissues - for example, the epidermis of a user, is shown. The apparatus 1 comprises a body 10 on which a user interface 20, comprising input/output devices, and a plurality of connectors 30, eight in the example considered, each adapted to receive a corresponding connection terminal 41 of an electrode 40 connected by an electric cable 45 to an opposite probe terminal 43, which is adapted to contact a portion of a biological tissue B, are visible.

The apparatus 1 further comprises an electronic board 50 contained in the body 10. The electronic board 50 comprises a control unit 51 and a signal generator 53 and, possibly, a memory 55.

The control unit 51 is operatively connected to the signal generator 53, to the memory 55, to the user interface 20 for providing and/or exchanging signals with them. In particular, the control unit 51 is adapted to implement one or more algorithms and/or procedures as described below. For example, the control unit 51 can comprise one or more among a microcontroller, a microprocessor, an ASIC, an FPGA and, possibly, one or more ancillary circuits, such as a circuit for generating a synchronization signal (clock), amplifiers for input/output signals, power supply circuitry, etc.

The signal generator 53 is operatively connected to the connectors 30 to apply and detect electrical signals associated with a portion of the biological tissue B - through the corresponding connector 30 and electrode 40 applied - or between a couple of portions of the biological tissue B.

Moreover, the electronic board 50 can comprise a communication module 60 coupled to the control unit 51 for exchanging data with an external unit, for example a server 70 through a communication network 80.

In use, one or more electrodes 40 are applied to respective portions of the biological tissue B to transmit them (electrical) stimulus signals.

In a preferred embodiment, the control unit 51 implements a stimulation procedure 900, which allows generating a stimulus signal S, preferably having constant values of voltage and current, and transmitting it to at least one electrode 40 connected to a respective connector 30.

Preferably, the stimulus signal S can be defined by an operator of the apparatus 1 or automatically, in order to bring a static potential V_{B} of the portion of biological tissue B to which the electrode 40 is applied to a target value selected by the operator of the apparatus 1.

For example, the operator selects (block 901), via the user interface 20, one or more operating parameters of the stimulus signal S to obtain a target value of the static potential V_{B} of the portion of biological tissue B. Advantageously, the operating parameters can be completely defined by the operator and/or the control unit 51 can be configured to provide a set of stimulus signals S selectable by the operator via the interface 20.

The control unit 51 is configured for receiving the operating parameters selected by the operator and, possibly, for defining further operating parameters necessary to generate the stimulus signal S and transmit them to the signal generator 53 (block 903). In the preferred embodiment, the control unit generates a digital stimulus signal S - for example, a constant value - based on the defined operating parameters which is then transmitted to the signal generator 53.

The digital stimulus signal S is converted (block 905) into an analog stimulus signal S by the signal generator 53 and is then applied, via the connector 30 and the electrode 40, to the portion of biological tissue B to which the electrode 40 has been applied. In particular, stimulus signal S is applied for a time sufficient to bring this portion of biological tissue B to the target value of the static potential V_{B}. For example, the signal generator 53 can comprise a DAC suitable for converting a digital signal provided at the input with a corresponding analog signal provided at the output.

In the preferred embodiment, the control unit 51 also implements an adjustment procedure 1000, which allows generating one or more (electrical) adjustment signals sₜₓ, with a time-varying trend, to at least one electrode 40 connected to a respective connector 30.

The control unit 51 determines the operating parameters P (block 1001) of one or more adjustment signals sₜₓ to be transmitted to a selected electrode 40 - and, therefore, to the corresponding portion of biological tissue B. In the preferred embodiment, the control unit 51 is configured for generating digital adjustment signals sₜₓ generated on the basis of operating parameters P. Preferably, the digital adjustment signals sₜₓ are also generated based on additional parameters A, for example entered by an operator via the user interface 20 or, alternatively, by suitable sensors coupled to the apparatus 1.

In the present document, the term 'operating parameters' comprises, but is not limited to, one or more of a voltage and/or electrical current value, a waveform, a frequency value, a phase, a duration, etc., of the electrical signal considered. For example, one or more sets of operating parameters P relating to the adjustment signal sₜₓ can be stored in the memory 55.

Otherwise, the term 'additional parameters' comprises, but is not limited to one or more indications on the position of the probe terminals 43 on the biological tissue B, indications of a subject treated by the apparatus - such as weight, height, sex, age, etc. - and environmental indications - such as temperature and atmospheric pressure, and the time of the day.

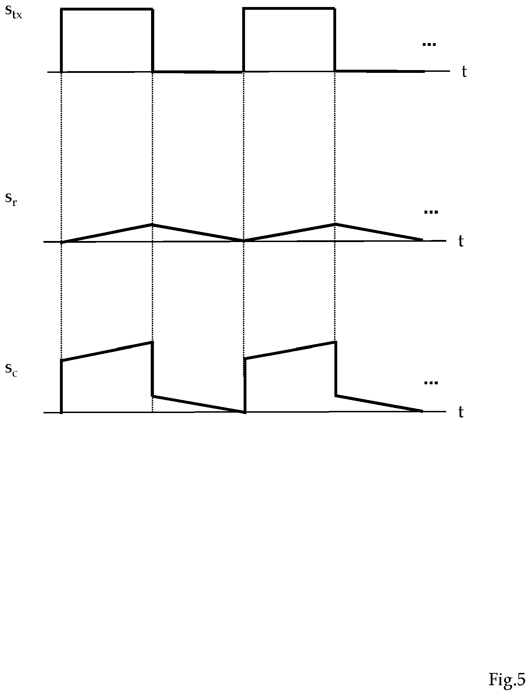

The signal generator 53 generates (block 1003) an analog adjustment signal sₜₓ by converting the digital adjustment signal sₜₓ. The adjustment signal sₜₓ propagates through the electrode 40 up to the portion of biological tissue B. For example, the DAC of the signal generator 53 converts the digital adjustment signal sₜₓ provided by the controller 51 into the corresponding analog adjustment signal sₜₓ, for example a square wave as shown in Figure 5, where the contribution of the stimulus signal S to the connector signal s_{c} and to the return signal sᵣ have been omitted.

The signal generator 53 also detects an analog connector signal s_{c} at the connector 30 crossed by the stimulus signal sₜₓ and converts it into a corresponding digital connector signal s_{c} which is provided to the control unit 51 (block 1005). For example, the signal detector comprises an ADC for converting the analog connector signal s_{c} into a corresponding digital connector signal s_{c} read by the control unit 51. In particular, the connector signal s_{c} corresponds to the combination between the adjustment signal sₜₓ and a return signal sᵣ, which is generated by a reflection of the stimulus signal sₜₓ due to a difference between the impedance of the electrode 40 and the impedance Z_{B} of the portion of biological tissue B to which the electrode 40 is applied. For example, the return signal sᵣ is a sawtooth signal and the connector signal s_{c} corresponds to the combination between the square wave and the sawtooth wave as shown in Figure 5.

The control unit 51 is configured for determining (block 1007) one or more operating parameters R of the return signal sᵣ and for comparing (decision block 1009) these latter with one or more sets of corresponding expected parameters T stored in the memory 55.

In particular, the operating parameters R of the return signal sᵣ are determined from the impedance Z_{B} of the portion of biological tissue B to which the selected electrode 40 is applied. The impedance Z_{B} in turn depends on a variable electrical potential v_{B} of the same portion of biological tissue B. This variable electrical potential v_{B} varies over time and depends on the distribution of ions in biological tissue B, which varies in space and time in accordance with chemical-physical reactions (typically Redox) that take place in the cells of the biological tissue B itself substantially without any break in continuity.

The expected parameters T of each set correspond, instead, to operating parameters of the return signal sᵣ. Studies carried out by the Applicant have determined that it is possible to deduce a chemical-physical condition of the portion of biological tissue B to which a given electrode 40 is applied, by analysing the return signal sᵣ generated by the reflection of a known adjustment signal sₜₓ. Therefore, the Applicant has determined the expected parameters T which correspond to an optimal condition of the biological tissue B, for example an impedance adaptation which allows a maximum transmission of the stimulus signal S and/or of the adjustment signal sₜₓ (for example in terms of transferred energy) and/or indicative of an optimal chemical-physical condition of the corresponding portion of biological tissue B based on criteria below.

In the preferred embodiment, the expected parameters T correspond to an optimal return signal sᵣ, defined on the basis of a response to the adjustment signal sₜₓ of a Hodgkin-Huxley model of the biological tissue B.

If the operating parameters R and the expected parameters T correspond (output branch Y of block 1009), the adjustment signal sₜₓ is optimized (block 1011) for the application to the corresponding portion of biological tissue B and is maintained for a period of predetermined time (for example, in the order of minutes). Preferably, the correspondence between the operating parameters R and the expected parameters T is met if each operating parameter R is contained within a tolerance range centred on the respective expected parameter T. In other words, the correspondence of the parameters is met when a difference (in module) between the operating parameters R and the expected parameters T is less than a threshold value - preferably, a respective threshold value can be defined and met for each parameter considered in the comparison with block 1009. Below, for example periodically, the control unit 51 brings the operation back to block 1005 to verify the optimization of the adjustment signal sₜₓ provided to the electrode 40.

If a discrepancy - greater than the threshold value - between the operating parameters R and the expected parameters T (output branch N of block 1009) is given, the control unit 51 modifies (block 1013) one or more operating parameters P of the adjustment signal sₜₓ to cancel, or at least to reduce within the threshold value, the discrepancy between the operating parameters R and the expected parameters T of the return signal sᵣ.

For example, the control unit 51 is configured for modifying the operating parameters P of the adjustment signal sₜₓ according to a relationship that is proportional to the entity of the detected difference.

The operation then returns to block 1003 to reiterate the steps described above until obtaining - at block 1011 - the adjustment signal sₜₓ optimized for the corresponding portion of biological tissue B to which the connector 40 is applied.

The adjustment procedure 1000 can be reiterated by the control unit during the operation of the apparatus 1 so as to provide an adjustment signal sₜₓ optimized for each electrode 40 sequentially or, alternatively, the control unit 51 can perform more instances of the adjustment procedure 1000 in order to provide a corresponding optimized adjustment signal sₜₓ to multiple electrodes 40 in parallel. In particular, adjustment signal sₜₓ varies dynamically over time to maintain the variable electrical potential v_{B} - or, in an equivalent manner, the impedance Z_{B} - indicative of the optimal chemical-physical condition for each portion of biological tissue B to which a corresponding electrode 40 is applied.

In a preferred embodiment, the operator can activate, through the user interface 20, a procedure for measuring 1100 reference potentials of the biological tissue B.

The signal generator 53 is brought by the control unit 51 to a measurement configuration in which it performs a high impedance measurement of the total electrical potential V_{BT} through a selected electrode 40 (block 1101). In the present document, total electrical potential V_{BT} means the combination - typically, the algebraic sum - of the static electrical potential V_{B} and of the variable electrical potential v_{B}.

Advantageously, the value of the measurement impedance is sized so as to allow a reading of very weak total electrical potentials V_{BT}, for example in the order of tens of millivolts, without deteriorating the chemical interactions at molecular and ionic level which take place in the biological tissue B and which determine the total electrical potentials V_{BT}. For example, the measurement impedance is sized so as to absorb a current of the order of Ampere peak or lower during the measurement of the total electrical potential V_{BT} of the connectors 40.

Preferably, the measurement of the total electrical potential V_{BT} is carried out with respect to an electrical potential provided by a reference electrode 40 applied to the biological tissue B, for example in a predefined portion of the biological tissue B - as a part of the umbilical region of a subject treated by means of the apparatus 1.

The measurement of the total electrical potential V_{BT} is converted by the signal generator 53 into a corresponding digital signal and transmitted to the control unit 51 (block 1103).

The control unit 51 then modifies (block 1105) the total electrical potential measurements V_{BT} made on the basis of the additional parameters A provided by the operator through the user interface 20. In this way, it is possible to determine with greater accuracy the actual chemical-physical condition of the biological tissue B offsetting the effects introduced by environmental conditions (e.g., ambient temperature), from the position of the electrodes and the subject being treated.

The modified total electrical potential measurements V_{BT} can be provided to the operator through the user interface 20, to the server 70 and/or stored in the memory 65 (block 1107). Preferably, the server 70 implements algorithms which allow identifying one or more portions of biological tissue B to which the stimulus procedure 900 and the adjustment procedure 1000 are to be applied to obtain a desired effect.

In addition, the server 70 can process the electrical potential measurements v_{B} and provide information relating to the condition of the biological tissue B which can be viewed through the user interface 20 (block 1109).

Thanks to the implementation of the procedures 900 and 1000 described above, the apparatus 1 is suitable for transmitting stimulus signals S and adjustment signals sₜₓ to the biological tissue B to obtain an antalgic procedure, in particular, based on an electrical stimulation with micro-currents in a particularly effective way.

In detail, the stimulation procedure 900 is implemented so as to bring the portion of biological tissue B, to which a respective electrode 40 is applied, to a constant electrical potential V_{B} having a value comprised between 0 V and -30 mV. The constant electrical potential V_{B} comprised within these values is indicative of an absence of algic aspects in the biological tissue B and/or in adjacent tissues - for example, epidermis and muscular tissues.

Moreover, the adjustment procedure 1000 allows the adjustment signals sₜₓ to be adapted dynamically in accordance with chemical-physical variations of the biological tissue B, so as to optimize the stimulation based on the actual conditions of the biological tissue B and consequently increase the efficiency of micro-current electrostimulation implemented by the apparatus 1.

Preferably, the antalgic procedure for the execution of the antalgic procedure uses electrodes 40 with a surface probe terminal 43 in the order of cm² such as for example comprised between 1 cm² and 5 cm². The Applicant has in fact determined that such an extension of the probe terminals 43 of these is particularly suitable for micro-current electrostimulation, in particular in the treatment of biological tissue B belonging to human subjects.

In the preferred embodiment, the control unit 51 can be configured for implementing an antalgic procedure 1200 which envisages the transmission of the stimulus signals S and sₜₓ through each electrode 40 in a sequential manner.

In detail, the control unit 51 is configured for applying the stimulation procedures 900 and those of adjustment 1000 in order to define a desired total electrical potential V_{BT} for the portion of biological tissue B to which a first electrode of the sequence is applied (block 1201). Once the control unit 51 verifies that the desired total electrical potential V_{BT} has been reached (decision block 1203), the control unit 51 selects (block 1205) the second electrode 40 in the sequence and reiterates the operations described above until obtaining a desired total electrical potential V_{BT} for the portion of biological tissue B associated with the second electrode 40.

The automated sequential operation implemented by the antalgic procedure 1200 is particularly effective in the treatment of algic aspects that propagate from a point of origin; for example, pain related to the sciatic nerve that develops from the lumbar region of a subject to his/her feet.

Therefore, the apparatus 1 can be used to administer an effective antalgic treatment adapting itself automatically to the peculiarities of the treated subject - thanks to the adjustment procedure 1000.

Tests carried out by the Applicant have established that the antalgic procedure 1200 implemented by the apparatus 1 allows each portion to be brought to the desired total electrical potential V_{BT} over a time in the order of minutes such as, for example, between 1 minute and 5 minutes. In addition, the attenuation of algic aspects obtained with the antalgic procedure 1200 derives from a corresponding reduction of the inflammatory mechanisms/states and not from a block of the transmission of painful information by interfering on the mechanisms of transmission of neurological pain information.

The apparatus 1 is also suitable for the implementation of a particularly effective transcutaneous electroacupuncture procedure 1300.

Preferably, the transcutaneous electroacupuncture procedure 1300 envisages using electrodes 40 with a surface probe terminal 43 in the order of mm² such as for example comprised between 0.5 mm² and 2 mm². The Applicant has in fact determined that such an extension of the probe terminals 43 is particularly suitable for replicating the effects of a treatment of acupuncture without using any needles or other tools intended to be driven into the biological tissue B.

In particular, the transcutaneous electroacupuncture procedure 1300 envisages performing the measurement procedure 1100. Preferably, the transcutaneous electroacupuncture procedure 1300 envisages measuring (block 1301) a potential at a plurality of predetermined points, for example 60 acupuncture points or "Shu" defined by the TCM.

The measurements made are then transmitted by the control unit 51 to the server 70 which processes these measurements in order to generate a report to be provided to the operator as described above (block 1303). Moreover, in the preferred embodiment, the server 70 identifies one or more acupuncture points on which to intervene through the procedure and enters this information in the report for the operator. For example, the server 70 highlights one or more points aligned on a corresponding meridian to be brought into an equilibrium state according to what is defined by the TCM.

In a preferred embodiment, the server 70 implements one or more algorithms suitable for recognizing a state of the acupuncture points and identifying an imbalance among the points along one or more meridians in accordance with the TCM on the basis of the measurements of total electrical potential V_{BT}.

On the basis of the information received through the user interface 20, the operator can arrange (block 1305) two or more electrodes 40 on the biological tissue B according to a specific scheme, for example, on portions of biological tissue B aligned along one of the meridians defined by the TCM signalled in the report provided by the server 70.

The control unit 51 is then configured for implementing a stimulation procedure 900 and one of adjustment 1000 to bring the portions of biological tissue B to which the electrodes 40 are applied to corresponding total electrical potentials V_{BT}.

In particular, the stimulation procedure 900 is implemented to apply a stimulus signal S to a corresponding portion of biological tissue B such as to impose a respective constant electrical potential V_{B}. In particular, studies carried out by the Applicant have shown that it is possible to identify values of constant electrical potential V_{B} - with positive or negative sign, according to the cases - which allow obtaining a chemical-physical condition corresponding to specific equilibrium conditions of the biological tissue B defined according to the TCM's criteria. For example, the operator selects the operating parameters of the stimulus signal S - for example, an electrical current intensity associated with the Stimulus signal - based on the information contained in the report provided by the server 70.

At the same time, the adjustment procedure 1000 is implemented to modify the variable electrical potentials v_{B} of the electrodes 40 applied to the biological tissue B so as to establish chemical-physical conditions of the biological tissue B allowing the operation of the apparatus 1 to be adapted to the peculiarities of the subject treated, increasing the efficacy of transdermal electroacupuncture.

The transcutaneous electroacupuncture procedure 1300 is performed for a time sufficient to bring the total electrical potentials V_{BT} of the portions of biological tissue B to which the electrodes 40 are applied to respective values which allow obtaining an equilibrium condition according to the TCM's criteria.

The invention thus conceived is susceptible to several modifications and variations, all falling within the scope of the inventive concept.

For example, the signal generator 53 can comprise one or more amplifiers for amplifying the electrical signals sₜₓ and s_{c}.

The signal generator 53 can be configured for transmitting the stimulus signals sₜₓ sequentially or, alternatively, in parallel through the electrodes 40. In the same way, the signal generator 53 can be configured for detecting sequentially or, in parallel, the connector signals s_{c}.

Again, signal generator 53 can be configured for determining the return signal sᵣ and transmit it to the control unit 51.

In particular, the control unit 51 can be configured for generating the stimulus signals sₜₓ so as to maintain the value of the electrical potentials v_{B} of all the portions of biological tissue B within a same range of values, at the same value or, otherwise, each at a respective value.

In an alternative embodiment, a device - for example, a computer, a tablet, a smartphone - can be connected to the apparatus 1 and implement an interface software usable in combination or as an alternative to the user interface 20. Furthermore, nothing prevents the control unit 51 from exploiting the computing power of the device to perform at least a part of the procedures described above.

In addition, the communication module 60 is adapted to establish a connection with the server 70 via a wired or radio frequency connection with the communication network 80. In addition or as an alternative, the control unit 51 can exchange data with the server 70 through the device connected to the apparatus 1.

Again, nothing prevents from manufacturing an electronic unit capable of directly performing the analysis of the parameters to provide information, to select and/or to propose sequences of stimulus signals, therefore, without having to connect to an external entity.

Moreover, the procedures described above may envisages applying a conductive gel between the probe terminals 43 of the electrodes and the biological tissue B to ensure a low resistivity, a stability and uniformity of the electrical contact between them. In one embodiment, the conductive gel comprises one or more active ingredients (for example a phytotherapeutic drug) suitable for the treatment of inflammatory and/or painful states. The Applicant has observed that the absorption of these active ingredients in the biological tissue B, is greatly improved by the electrical stimulation applied through the electrodes. In other words, the combined use of electrostimulation and conductive gel comprising active ingredients increases the efficacy of the latter. This makes it possible to combine the antalgic and/or transdermal electroacupuncture procedures with a local administration of active ingredients in order to obtain a particularly effective and long-lasting treatment of painful conditions.

In practice, the materials used, as well as the contingent shapes and sizes, can be whatever according to the requirements without for this reason departing from the scope of protection of the following claims.

## Claims

1. An apparatus (1) for electrostimulation of biological tissues, comprising:
- a signal generator (53) adapted to generate an electrical signal (sₜₓ),
- an electrode (40) adapted to be applied to a biological tissue and electrically connected to the signal generator (53) to receive the electrical signal generated by the signal generator (53),
- a control unit (51) operatively connected to the signal generator (53) to adjust parameters of the generated electrical signal and to read the parameters of an electrical signal returning from the electrode (40),
wherein the control unit (51) is configured for
a) determining (1001) the parameters of the electrical signal (sₜₓ),
b) measuring (1005) a return electrical signal (sᵣ) returning from the electrode (40) and generated by a reflection of the electrical signal (sₜₓ) due to a difference between the impedance of the electrode (40) and the impedance (Z_{B}) of the portion of biological tissue (B) to which the electrode (40) is applied,
c) comparing (1009) at least one parameter of the return electrical signal (sᵣ) with a corresponding parameter of an expected return signal, and
d) if one or more of the parameters of the return electrical signal (sᵣ) differ from the corresponding parameters of the expected return signal beyond a threshold value, then the control unit (51) is configured for modifying (1013) the parameters of the electrical signal (sₜₓ) and repeating steps b) to d).

2. The apparatus (1) according to claim 1, wherein the parameters comprise a voltage value, an electrical current value, a waveform, a frequency value, a phase, a duration of the respective electrical signal (sₜₓ, sᵣ).

3. The apparatus (1) according to claim 1, wherein the control unit (51) is configured for generating the parameters of the electrical signal (sₜₓ) based on additional parameters comprising at least one among an ambient temperature, an atmospheric pressure, and a time of the day.

4. The apparatus (1) according to claim 1, wherein the control unit (51) is configured for generating the parameters of a second electrical signal (S) suitable for bringing a portion of biological tissue (B) onto which an electrode is applied (40) to a predetermined electrical potential (V_{B}).

5. The apparatus (1) according to claim 1, wherein the control unit (51) is configured for generating (1201) the parameters of a plurality of electrical signals (sₜₓ) each of which is sequentially transmitted by a respective electrode (40), and wherein the control unit (51) is configured for generating (1203,1205) the parameters of an electrical signal (sₜₓ) when the parameters of the return electrical signal (sᵣ) associated with a previous electrical signal (sₜₓ) differ from the corresponding parameters of the expected return signal within the threshold value.

6. The apparatus (1) according to claim 1, further comprising a user interface (20), and
wherein the control unit (51) is configured for
- measuring (1100) an electrical potential (V_{BT}, V_{B}, v_{B}) by means of the electrode (40), and
- displaying (1109) information based on the electrical potential (V_{B}, V_{B}, v_{B}) by means of the electrode (40) through the user interface (20).

7. The apparatus (1) according to claim 6, wherein the control unit (51) is configured for
- transmitting (1107) to an external unit (70) electrical potential measurements (V_{B}, V_{B}, v_{B}) made by means of at least one electrode (40),
- receiving (1109) information from the external unit (70), such information comprising an indication of one or more electrodes (40) to which the electrical signal (sₜₓ) is to be applied identified based on performed electrical potential measurements (V_{BT}, V_{B}, v_{B}).

8. The apparatus (1) according to claim 6 or 7, further comprising a reference electrode (40), and
wherein the control unit (51) is configured for measuring the electrical potential (V_{BT}, V_{B}, v_{B}) between the electrode (40) and the reference electrode (40).

9. The apparatus (1) according to claim 1, wherein the electrodes (40) comprise probe terminals (43) suitable for contacting a biological tissue (B), the probe terminals (43) having a contact surface of extension comprised between 0.5 mm² and 2 mm² or comprised between 1 cm² and 5 cm².

## Patentansprüche

1. Vorrichtung (1) zur Elektrostimulation von biologischem Gewebe, umfassend:
- einen Signalgenerator (53), der zur Erzeugung eines elektrischen Signals (sₜₓ) eingerichtet ist,
- eine Elektrode (40), die zur Anwendung an einem biologischen Gewebe eingerichtet ist und elektrisch mit dem Signalgenerator (53) verbunden ist, um das von dem Signalgenerator (53) erzeugte elektrische Signal zu empfangen,
- eine Steuereinheit (51), die betriebsmäßig mit dem Signalgenerator (53) zum Einstellen von Parametern des erzeugten elektrischen Signals und zum Lesen der Parameter eines von der Elektrode (40) zurückkommenden elektrischen Signals verbunden ist, wobei
die Steuereinheit (51) eingerichtet ist für das
a) Bestimmen (1001) der Parameter des elektrischen Signals (sₜₓ),
b) Messen (1005) eines elektrischen Rücklaufsignals (sᵣ), das von der Elektrode (40) zurückkommt und durch eine Reflexion des elektrischen Signals (sₜₓ) aufgrund einer Differenz zwischen der Impedanz der Elektrode (40) und der Impedanz (Z_{B}) des Teils des biologischen Gewebes (B) erzeugt wird, an dem die Elektrode (40) angewendet wird,
c) Vergleichen (1009) mindestens eines Parameters des elektrischen Rücklaufsignals (sᵣ) mit einem entsprechenden Parameter eines erwarteten Rücklaufsignals, und
d) wenn einer oder mehrere der Parameter des elektrischen Rücklaufsignals (sᵣ) von den entsprechenden Parametern des erwarteten Rücklaufsignals jenseits eines Schwellenwerts abweicht, dann ist die Steuereinheit (51) zum Ändern (1013) der Parameter des elektrischen Signals (sₜₓ) (1013) und zum Wiederholen der Schritte b) bis d) eingerichtet.

2. Vorrichtung (1) nach Anspruch 1, wobei die Parameter einen Spannungswert, einen elektrischen Stromwert, eine Wellenform, einen Frequenzwert, eine Phase, eine Dauer des jeweiligen elektrischen Signals (sₜₓ, sᵣ) umfassen.

3. Vorrichtung (1) nach Anspruch 1, wobei die Steuereinheit (51) zum Erzeugen der Parameter des elektrischen Signals (sₜₓ) auf der Basis von zusätzlichen Parametern eingerichtet ist, die mindestens einen aus einer Umgebungstemperatur, einem atmosphärischen Druck und einer Tageszeit umfassen.

4. Vorrichtung (1) nach Anspruch 1, wobei die Steuereinheit (51) eingerichtet ist, um die Parameter eines zweiten elektrischen Signals (S) zu erzeugen, das geeignet ist, einen Teil des biologischen Gewebes (B), auf dem eine Elektrode angewendet wird (40), auf ein vorbestimmtes elektrisches Potential (V_{B}) zu bringen.

5. Vorrichtung (1) nach Anspruch 1, wobei die Steuereinheit (51) zum Erzeugen (1201) der Parameter einer Vielzahl von elektrischen Signalen (sₜₓ) eingerichtet ist, von denen jedes sequentiell von einer betreffenden Elektrode (40) übertragen wird, und wobei die Steuereinheit (51) zum Erzeugen (1203, 1205) der Parameter eines elektrischen Signals (sₜₓ) eingerichtet ist, wenn die Parameter des elektrischen Rücklaufsignals (sᵣ), das einem vorherigen elektrischen Signal (sₜₓ) zugeordnet ist, von den entsprechenden Parametern des erwarteten Rücklaufsignals innerhalb des Schwellenwerts abweichen.

6. Vorrichtung (1) nach Anspruch 1, ferner aufweisend eine Benutzerschnittstelle (20),
und
wobei die Steuereinheit (51) eingerichtet ist für das
- Messen (1100) eines elektrischen Potentials (V_{BT}, V_{B}, v_{B}) mit Hilfe der Elektrode (40), und
- Anzeigen (1109) von Informationen basierend auf dem elektrischen Potential (V_{B}, V_{B}, v_{B}) mittels der Elektrode (40) über die Benutzerschnittstelle (20).

7. Vorrichtung (1) nach Anspruch 6, wobei die Steuereinheit (51) eingerichtet ist zum
- Übertragen (1107) von elektrischen Potentialmessungen (V_{B}, V_{B}, v_{B}), die mittels mindestens einer Elektrode (40) vorgenommen wurden, an eine externe Einheit (70),
- Empfangen (1109) von Informationen von der externen Einheit (70), wobei diese Informationen eine Angabe von einer oder mehreren Elektroden (40) umfassen, an die das elektrische Signal (sₜₓ) angelegt werden soll, die auf der Grundlage von durchgeführten elektrischen Potentialmessungen (V_{BT}, V_{B}, v_{B}) identifiziert wird.

8. Vorrichtung (1) nach Anspruch 6 oder 7, ferner aufweisend eine Referenzelektrode (40), und
wobei die Steuereinheit (51) zur Messung des elektrischen Potentials (V_{BT}, V_{B}, v_{B}) zwischen der Elektrode (40) und der Referenzelektrode (40) eingerichtet ist.

9. Vorrichtung (1) nach Anspruch 1, wobei die Elektroden (40) Sondenanschlüsse (43) umfassen, die für den Kontakt mit einem biologischen Gewebe (B) geeignet sind, wobei die Sondenanschlüsse (43) eine Kontaktfläche mit einer Ausdehnung zwischen 0,5 mm² und 2 mm² oder zwischen 1 cm² und 5 cm² umfasst.

## Revendications

1. Appareil (1) pour une électrostimulation de tissus biologiques, comprenant :
- un générateur de signal (53) conçu pour générer un signal électrique (sₜₓ),
- une électrode (40) conçue pour être appliquée sur un tissu biologique et connectée électriquement au générateur de signal (53) pour recevoir le signal électrique généré par le générateur de signal (53),
- une unité de commande (51) connectée de manière fonctionnelle au générateur de signal (53) pour ajuster des paramètres du signal électrique généré et pour lire les paramètres d'un signal électrique revenant de l'électrode (40),
l'unité de commande (51) étant configurée pour
a) déterminer (1001) les paramètres du signal électrique (sₜₓ),
b) mesurer (1005) un signal électrique de retour (sᵣ) revenant de l'électrode (40) et généré par une réflexion du signal électrique (sₜₓ) due à une différence entre l'impédance de l'électrode (40) et l'impédance (Z_{B}) de la partie de tissu biologique (B) sur laquelle l'électrode (40) est appliquée,
c) comparer (1009) au moins un paramètre du signal électrique de retour (sᵣ) à un paramètre correspondant d'un signal de retour attendu, et
d) si un ou plusieurs des paramètres du signal électrique de retour (sᵣ) diffèrent des paramètres correspondants du signal de retour attendu au-delà d'une valeur de seuil, alors l'unité de commande (51) est configurée pour modifier (1013) les paramètres du signal électrique (sₜₓ) et répéter les étapes b) à d).

2. Appareil (1) selon la revendication 1, dans lequel les paramètres comprennent une valeur de tension, une valeur de courant électrique, une forme d'onde, une valeur de fréquence, une phase, une durée du signal électrique respectif (sₜₓ, sᵣ).

3. Appareil (1) selon la revendication 1, dans lequel l'unité de commande (51) est configurée pour générer les paramètres du signal électrique (sₜₓ) sur la base de paramètres supplémentaires comprenant au moins l'une parmi une température ambiante, une pression atmosphérique et une heure de la journée.

4. Appareil (1) selon la revendication 1, dans lequel l'unité de commande (51) est configurée pour générer les paramètres d'un second signal électrique (S) approprié pour amener une partie de tissu biologique (B), sur laquelle une électrode (40) est appliquée, à un potentiel électrique prédéterminé (V_{B}).

5. Appareil (1) selon la revendication 1, dans lequel l'unité de commande (51) est configurée pour générer (1201) les paramètres d'une pluralité de signaux électriques (stx) dont chacun est séquentiellement transmis par une électrode respective (40), et dans lequel l'unité de commande (51) est configurée pour générer (1203, 1205) les paramètres d'un signal électrique (stx) lorsque les paramètres du signal électrique de retour (sr) associé à un signal électrique précédent (stx) diffèrent des paramètres correspondants du signal de retour attendu dans les limites de la valeur de seuil.

6. Appareil (1) selon la revendication 1, comprenant en outre une interface utilisateur (20),
et
dans lequel l'unité de commande (51) est configurée pour
- mesurer (1100) un potentiel électrique (VBT, VB, vB) au moyen de l'électrode (40), et
- afficher (1109) des informations sur la base du potentiel électrique (VB, VB, vB) au moyen de l'électrode (40) par l'intermédiaire de l'interface utilisateur (20).

7. Appareil (1) selon la revendication 6, dans lequel l'unité de commande (51) est configurée pour
- transmettre (1107), à une unité externe (70), des mesures de potentiel électrique (VB, VB, vB) réalisées au moyen d'au moins une électrode (40),
- recevoir (1109) des informations en provenance de l'unité externe (70), de telles informations comprenant une indication d'une ou plusieurs électrodes (40) à laquelle ou auxquelles le signal électrique (stx) doit être appliqué, identifiées sur la base de mesures de potentiel électrique réalisées (VBT, VB, vB).

8. Appareil (1) selon la revendication 6 ou 7, comprenant en outre une électrode de référence (40), et
dans lequel l'unité de commande (51) est configurée pour mesurer le potentiel électrique (VBT, VB, vB) entre l'électrode (40) et l'électrode de référence (40).

9. Appareil (1) selon la revendication 1, dans lequel les électrodes (40) comprennent des bornes de sonde (43) appropriées pour entrer en contact avec un tissu biologique (B), les bornes de sonde (43) ayant une surface de contact d'extension comprise entre 0,5 mm² et 2 mm² ou comprise entre 1 cm² et 5 cm².
